# EUROPÄISCHE PATENTSCHRIFT

(11) **EP 0 766 556 B1**
(45) Veröffentlichungstag und Bekanntmachung des Hinweises auf die Patenterteilung: **18.08.1999**
(21) Anmeldenummer: 95921827.2
(22) Anmeldetag: 03.06.1995
(51) Int. Cl.: A61K 9/70

(54) **VOLUMEN-EXPANDIERBARE, ALS WIRKSTOFFTRÄGER, INSBESONDERE ZUR ORALEN ANWENDUNG GEEIGNETE FLÄCHIGE ANWENDUNGSFORM**
VOLUME-EXPANDABLE FLAT ARTICLE SUITABLE FOR USE AS AN ACTIVE SUBSTANCE CARRIER, IN PARTICULAR FOR ORAL USE
FORME GALENIQUE PLATE EXPANSIBLE POUVANT ETRE UTILISEE COMME EXCIPIENT, NOTAMMENT POUR ADMINISTRATION PAR VOIE ORALE

(30) Priorität: 07.06.1994 DE 4419824
(43) Veröffentlichungstag der Anmeldung: 09.04.1997
(73) Patentinhaber: LTS LOHMANN Therapie-Systeme GmbH, 56567 Neuwied (DE)
(72) Erfinder: BECHER, Frank, 56072 Koblenz (DE)
(74) Vertreter: Flaccus, Rolf-Dieter, Dr.
(86) Internationale Anmeldenummer: EP9502120
(87) Internationale Veröffentlichungsnummer: WO9533452

(56) Entgegenhaltungen:
- EP-A- 0 460 588
- FR-A- 2 571 253

## Beschreibung

Die Erfindung betrifft eine volumen-expandierbare, flächenförmige, als Wirkstoffträger insbesondere zur oralen Anwendung geeignete Anwendungsform in Form einer eine Mischung von Füllstoffen, Reinigungs-, Pflege- oder Wirkstoffen, Filmbildnern und Weichmachern enthaltenden Folie.

Bekannt sind flächenförmige Substrate, insbesondere zur oralen Anwendung. Durch derartige Substrate werden eine Reihe von Vorteilen erreicht, insbesondere geringes Gewicht und geringes Volumen, die eine Reihe von Vorteilen im Rahmen einer ökologischen Gesamtbilanz bieten und beispielsweise nur geringe Verpackung und Stapelraum beanspruchen

Die DE 40 18 247 beschreibt eine folienförmige, einzeln dosierte, in Wasser schnell zerfallende Darreichungsformen von Arzneimitteln, Süßwaren, anderen Lebensmitteln, Kosmetika zur oralen Anwendung oder Einnahme wobei diese eine Grundmasse auf einem Träger enthält oder trägerfrei aus der Grundmasse besteht. Diese ist nach einem Verfahren mit folgenden Schritten herstellbar:
a) Filmbildner, Gelbildner, Wirkstoff und Füllstoff werden trocken innig vorgemischt;
b) die Vormischung wird unter Zusatz von bis zu 30 Gew.-% eines polaren Lösungsmittels unter Rühren in eine streichfähige Konsistenz gebracht und
c) die so gewonnene Masse wird auf eine Trennfolie in einer Schichtdicke zwischen 0,003 bis 4 mm, vorzugsweise 20 bis 400 µm und besonders bevorzugt 70 bis 150 µm aufgetragen;
d) das polare Lösungsmittel wird entfernt, und zwar bis auf eine zum Fließfähigmachen der Masse erforderlichen Teilmenge.

Bei derartigen folienförmigen Darreichungsformen wird als Nachteil empfunden, daß durch die Kleinheit sowie Leichtigkeit die Handhabung erschwert ist. Wenn sich derartige Nachteile vor der Entnahme aus einer geeigneten Spenderpackung überwinden lassen, so bleibt doch noch das Problem, daß das entnommene Produkt nicht handhabungsgerecht ist. Eine zur Abhilfe mögliche Ausstattung des Produktes mit einer klebrigen Oberfläche ergibt den anderen Nachteil, daß damit die Handhabung vor bzw. bei Entnahme aus einer Packung oder einem Spender erschwert ist. Eine Abhilfe durch geeignete Abdeckfolien ergibt den weiteren Nachteil, daß hierdurch Vorteile bezüglich geringem Gewicht, unproblematischer Entsorgung und Herstellungskosten durch die zusätzlichen Arbeitsschritte kompensiert werden.

Der Erfindung liegt die Aufgabe zugrunde, solche schwierig handhabbaren, flächenförmigen und kleinen Substrate bzw. Anwendungsformen zwecks besserer Handhabung unter Vermeidung der vorgenannten Schwierigkeiten und technischen Grenzen zu verbessern.

Die Lösung der Aufgabe gelingt bei einem volumen-expandierbaren, flächenförmigen, als Wirkstoffträger insbesondere zur oralen Anwendung vorgesehenen Anwendungsform der eingangs genannten Art mit der Erfindung dadurch, daß diese Anteile eines hochsaugfähigen Hydrogelbildners aufweist, der bei Wasserzutritt quillt und ein mehrfaches seines ursprüngliches Volumens annimmt.
Mit Vorteil wird hierdurch erreicht, daß das Substrat bzw. die Anwendungsform bei Hinzutreten eines flüssigen Mediums innerhalb kürzester Zeit ihr Volumen wesentlich vergrößert und dabei auch eine gewisse Klebrigkeit erreicht. In diesem Zusammenhang wird auch die Tatsache genutzt, daß in einem breiten Anwendungsfeld für diese Substrate die Berührung mit Feuchtigkeit bei der Anwendung erwünscht und erforderlich ist. Dabei wird erfindungsgemäß die Volumenvergrößezung durch den Einsatz geeigneter Quellstoffe erreicht, wie sie beispielsweise in Hygieneprodukten wie Windeln, Einlagen, bei der Monatshygiene etc. als sogenannte Absorbent Polymere bekannt sind. Darunter werden quellfähige Substanzen verstanden, die wässrige Flüssigkeiten aufnehmen und sich spontan mit diesen zu einem relativ stabilen Gel verbinden. Sie sind in der Lage, ein Vielfaches ihres Gewichtes an wäßrigen Flüssigkeiten zu absorbieren und dauerhaft festzuhalten. Das so entstandene Gel weist kettenförmige Moleküle auf, die zu einem dreidimensionalen Netzwerk verknüpft und in ein flüssiges Medium eingebettet sind.

Eine Ausgestaltung sieht vor, daß die Folie mit hochsaugfähigem Hydrogelbildner beschichtet ist.
Der hochsaugfähige Hydrogelbildner kann aber auch als Suspensionsbestandteil in die Folie eingebracht sein.
Der hochsaugfähige Hydrogelbildner ist ein Polper und enthält vorzugsweise schwervernetzte Carboxyvinylcopolymere und/oder quervernetzte Polyvinylpyrrolidone.
Weiterhin kann ein zusätzlicher Effekt noch dadurch erreicht werden, daß das Polymer bei Zutritt von Wasser aufschäumende Stoffe enthält. Solche Stoffe können Natriumbicarbonat und Zitronen- oder Ascorbinsäure oder andere physiologisch unbedenkliche Säure sein. Bei Verwendung dieser Schäume kann das Entstehen der Volumenvergrößerung gesteuert auch dazu beitragen, den fallweise erwünschten raschen Zerfall des flächenförmigen Systems noch weiter zu beschleunigen.
Dieser Effekt läßt sich insbesondere positiv bei einer Zubereitung zum Zähneputzen, einer "Zahnputzoblette" einsetzen. Bei dieser ist eine Volumenvergrößerung und Klebrigkeit nach der Entnahme besonders erwünscht. Dadurch kann die Zubereitung ähnlich einer Zahnpasta auf die Zahnbürste aufgebracht werden. Die Gefahr des Heruntergleitens wird dabei entscheidend verringert.
Weiterhin kann eine Variante vom Fachmann für solche Anwendungsformen vorgesehen sein, die geschluckt werden. Hier kann die äußere Schicht eines Hydrogels so beschaffen sein, daß sie problemlos aufgrund des Einsatzes von wasseraufnehmenden Quellstoffen die Speiseröhre passiert, um in einem späteren Stadium schnell zu zerfallen.

### Beispiel für ein erfindungsgemäßes flächenförmiges System

24 g einer 17%igen (g/g) Lösung von Polyvinylpyrrolidon (z.B. Kollidon® 90) in Ethanol werden mit 5 g eines quervernetzten Carboxyvinylcopolymers (Aquakeep® 10 SH) zu einer viskosen Suspension luftblasenfrei gerührt. Die Masse wird mit Hilfe eines Filmziehrahmens in einer Spaltbreite von 500 µm auf siliconisiertem Papier ausgestrichen. Es wird anschließend 5 Minuten bei 70 °C getrocknet. Der entstehende Film wird vom Trennpapier abgezogen und weist gegenüber dem Vergleichsbeispiel größeres Volumen und verbesserte Formstabilität auf bei unwesentlich verlängerter Zerfallszeit.

### Vergleichsbeispiel

30 g einer 17%igen (g/g) Lösung von Polyvinylpyrrolidon (z.B. Kollidon® 90) in Ethanol werden mit Hilfe eines Filmziehrahmens in einer Spaltbreite von 500 µm auf siliconisiertem Papier ausgestrichen. Es wird anschließend 5 Minuten bei 70 °C getrocknet. Der entstehende Film wird vom Trennpapier abgezogen.

### Weitere Substanzen

- Füllstoffe: z.B. Calciumsulfat, -carbonat, -phosphat, Silicagel
- Wirkstoffe: Natriumfluorid, Natriummonofluorphosphat
- Schaumbildner: Natriumdodecysulfonat, Neutraltenside
- Filmbildner: Polyvinylalkohol, Polyvinylpyrrolidon
- Aromen
- Weichmacher: Polyethylenglycole, Glycerin
- Süßstoffe: Saccharose, Cyclamate, Saccharin.

## Patentansprüche

1. Volumen-expandierbare flächenförmige, als Wirkstoffträger insbesondere zur oralen Anwendung geeignete Anwendungsform in Form einer eine Mischung von Füllstoffen, Reinigungs-, Pflege- oder Wirkstoffen, Filmbildnern und Weichmachern enthaltenden Folie, dadurch gekennzeichnet, daß diese Anteile eines hochsaugfähigen Hydrogelbildners aufweist, der bei Wasserzutritt quillt und eine Mehrfaches seines ursprünglichen Volumens annimmt.

2. Anwendungsform nach Anspruch 1, dadurch gekennzeichnet, daß die Folie mit hochsaugfähigem Hydrogelbildner beschichtet ist.

3. Anwendungsform nach Anspruch 1, dadurch gekennzeichnet, daß der hochsaugfähige Hydrogelbildner als Suspensionsbestandteil in die Folie eingebracht ist.

4. Anwendungsform nach Anspruch 1, dadurch gekennzeichnet, daß der hochsaugfähige Hydrogelbildner ein Polymer ist.

5. Anwendungsform nach Anspruch 4, dadurch gekennzeichnet, daß der Hydrogelbildner quervernetzte Carboxyvinylcopolymere und/oder quervernetzte Polyvinylpyrrolidone enthält.

6. Anwendungsform nach einem oder mehreren der Ansprüche 1-5, dadurch gekennzeichnet, daß das Polymer bei Zutritt von Wasser aufschäumende Stoffe enthält.

7. Anwendungsform nach Anspruch 6, dadurch gekennzeichnet, daß die aufschäumenden Stoffe Natriumbicarbonat mit Zitronen- oder Ascorbinsäure oder andere physiologisch unbedenkliche Säuren enthalten.

8. Anwendungsform nach einem oder mehreren der Ansprüche 1-7, dadurch gekennzeichnet, daß das Polymer oral verträgliche Kleber wie Stärkekleber enthält.

## Claims

1. A volume-expandable, sheet-like application form suitable as active substance carrier, in particular for oral application, in the form of a film comprising a mixture of fillers, cleansers, cosmetics, or active substances, film formers, and softeners, characterized in that it has portions of a highly absorbent hydrogel former which swells on access of water and assumes several times its original volume.

2. The application form according to claim 1 characterized in that the film is coated with the highly absorbent hydrogel former.

3. The application form according to claim 1 characterized in that the highly absorbent hydrogel former is incorporated into the film as a suspension component.

4. The application form according to claim 1 characterized in that the highly absorbent hydrogel former is a polymer.

5. The application form according to claim 4 characterized in that the hydrogel former comprises cross-linked carboxyvinyl copolymers and/or cross-linked polyvinyl pyrrolidones.

6. The application form according to one or several of claims 1-5 characterized in that the polymer comprises effervescent substances on access of water.

7. The application form according to claim 6 characterized in that the effervescent substances comprise sodium bicarbonate with citric or ascorbic acid, or other physiologically acceptable acids.

8. The application form according to one or several of claims 1-7 characterized in that the polymer comprises adhesives, such as starch adhesives, that are well tolerated orally.

## Revendications

1. Forme d'application plate expansible en volume appropriée comme support de principe actif en particulier pour l'application par voie orale, sous la forme d'une feuille contenant un mélange de matières de charge, de substances de purification, de substances curatives ou de principes actifs, d'agents filmogènes et de plastifiants, caractérisée en ce qu'elle présente des fractions d'un formateur d'hydrogel manifestant une capacité d'aspiration élevée, qui gonfle lorsque l'eau y accède et absorbe un multiple de son volume initial.

2. Forme d'application selon la revendication 1, caractérisée en ce que la feuille est enduite d'un formateur d'hydrogel manifestant une capacité d'aspiration élevée.

3. Forme d'application selon la revendication 1, caractérisée en ce que le formateur d'hydrogel manifestant une capacité d'aspiration élevée est incorporé dans la feuille à titre de constituant de suspension.

4. Forme d'application selon la revendication 1, caractérisée en ce que le formateur d'hydrogel manifestant une capacité d'aspiration élevée est un polymère.

5. Forme d'application selon la revendication 4, caractérisée en ce que le formateur d'hydrogel contient des copolymères carboxyvinyliques soumis à une réticulation transversale et/ou des polyvinylpyrrolidones soumises à une réticulation transversale.

6. Forme d'application selon une ou plusieurs des revendications 1 à 5, caractérisée en ce que le polymère contient des substances se transformant en mousse lorsque l'eau y accède.

7. Forme d'application selon la revendication 6, caractérisée en ce que les substances se transformant en mousse contiennent du bicarbonate de sodium avec de l'acide citrique ou de l'acide ascorbique ou encore d'autres acides physiologiquement acceptables.

8. Forme d'application selon une ou plusieurs des revendications 1 à 7, caractérisée en ce que le polymère contient des adhésifs compatibles par voie orale tels que des adhésifs contenant de l'amidon.
